# EUROPEAN PATENT SPECIFICATION

(11) **EP 2 029 629 B1**
(45) Date of publication and mention of the grant of the patent: **06.01.2010**
(21) Application number: 07729301.7
(22) Date of filing: 21.05.2007
(51) Int. Cl.: C08B 37/00, A61L 27/52, A61K 47/36

(54) **HYDROGELS OF POLYSACCHARIDE MIXTURES FOR TISSUE ENGINEERING AND AS CARRIERS OF ACTIVE COMPOUNDS**
HYDROGELE VON POLYSACCHARIDMISCHUNGEN FÜR DAS GEWEBE-ENGINEERING UND ALS WIRKSTOFFTRÄGER
HYDROGELS DE MÉLANGES DE POLYSACCHARIDES UTILISÉS EN GÉNIE TISSULAIRE ET EN TANT QUE VECTEURS DE COMPOSÉS ACTIFS

(30) Priority: 22.05.2006 IT PD20060203
(43) Date of publication of application: 04.03.2009
(73) Proprietor: Universita'Degli Studi di Trieste, I-34127 Trieste (IT)
(72) Inventor: PAOLETTI, Sergio, I-34136 Trieste (IT); DONATI, Ivan, 33039 Sedegliano (IT); MARSICH, Eleonora, I-34148 Trieste (IT)
(74) Representative: Gervasi, Gemma
(86) International application number: PCT/EP2007/054857
(87) International publication number: WO 2007/135114

(56) References cited:
- US-A- 5 620 706
- DONATI I ET AL: "The aggregation of pig articular chondrocyte and synthesis of extracellular matrix by a lactose-modified chitosan" BIOMATERIALS, ELSEVIER SCIENCE PUBLISHERS BV., BARKING, GB, vol. 26, no. 9, March 2005 (2005-03), pages 987-998, XP004568186 ISSN: 0142-9612 cited in the application

## Description

### Field of the invention

The present invention relates to hydrogels (or 3D matrices) obtainable from aqueous solutions of mixtures of acid polysaccharides and derivatives of basic polysaccharides, such as oligosaccharide derivatives of chitosan, suitably gelled with gelling agents and to their use in the biomedical field.

### State of the art

Polysaccharides, being generally biocompatible polymers, are extensively studied and have been used for some time for applications in the biomedical field as carriers of both biologically active compounds and biological matter such as cells for tissue engineering. Said process can be obtained, as is generally known by an expert in the field, by encapsulation or microencapsulation, i.e. inclusion of the biological material to be carried, within systems consisting of three-dimensional polymer matrices of the material itself. The characteristic which renders the polysaccharides suited to microencapsulation is their known capacity to form, in aqueous solution and under specific conditions, hydrogels which are in every respect three-dimensional polymer matrices. In particular, tissue engineering is a field in which the use of polysaccharides for the purposes of encapsulating cells is still subject to extensive research, being one of the most innovative aspects of biotechnological research. This technique arise from the need to move from replacement type transplant surgery to regenerative type surgery with biomaterials which favour regrowth of the same tissue cells, in order to have structural and physiological renewal of the original tissue as well as the complete recovery of metabolic activity and physical and functional integration with the surrounding tissue of the new tissue generated from the implanted cells.

There are a number of areas of application of tissue engineering and for certain therapeutic fields this approach can depend upon a consolidation of experience (for example in artificial skin). The technological progress in biotechnology fields has enabled a vast development in tissue engineering, including poorly explored areas. These certainly include the use of tissue engineering for the therapy of debilitating articular cartilage pathologies.

In this therapeutic field, a significant problem, additional to the more well-known problem of developing, within the sphere of cartilage regeneration surgery, biocompatible systems capable of recreating the optimum spatial and metabolic situation for cell growth and proliferation, is that of providing methods for *in vitro* culture of chondrocytes which allows their maintenance and expansion prior to the actual transplant itself. Furthermore, chondrocyte cultures are the most potent instrument for studying the molecular processes that accompany differentiation processes and the metabolic and functional modifications in physiological conditions or associated with pathological situations. The principal limitation to the use of chondrocyte cultures is that these cells, after being isolated from their matrix, show a marked tendency to de-differentiate into fibroblasts. Factors associated with, or which favour, the differentiation process are principally: culture systems in adhesion, low cellular density, presence of pro-inflammatory factors such a cytokines, cell immortalization. Under these conditions, cells rapidly lose, after a few days, their rounded shape typical of the chondrocyte phenotype and assume an elongated shape typical of fibroblasts. The modification of phenotype accompanies the down-regulated expression of specific chondrocyte markers, such as collagen II and collagen X, and high molecular weight proteoglycans such as aggrecan, together with the simultaneous increase in expression of collagen I and low molecular weight proteoglycans, such as biglycan or decorin. To avoid or anyhow limit the chondrocyte differentiation process, great effort has been made over the last 20 years to establish effective culture methods. These mainly comprise culture systems within (and not simply adhesion on) 3D alginate, agarose or collagen matrices. Although these approaches can improve maintenance of the chondrocyte phenotype, within them the cells show a very low growth and replication rate, at the expense of the amount of cellular material hence available.

One of the most utilized materials for entrapping cells within microcapsules, as.will be seen later, is alginate. The term alginate describes a family of polysaccharides produced from algae and bacteria (Sabra W. et al. Appl. Microbiol. Bíotechnol., 2001, 56, 315-25 ). It is composed of β-D-mannuronic acid and α-L-guluronic acid, joined through 1→4 bonds arranged in block structures along the polysaccharide chain. Due to the presence of carboxyl groups, the alginate is a polyanion at physiological pH, as are other polysaccharides with carboxyl groups. Furthermore, this polysaccharide is totally biocompatible (Uludag H. et al. Adv. Drug Deliv. Rev., 2000, 42, 29-64) while its most important physical characteristic for application in the industrial and biomedical field is linked to its capacity to form hydrogels on contact with solutions containing divalent ions, typically calcium. In this respect, the simple treatment of a concentrated alginate solution with said ions leads to the instantaneous formation of a hydrogel. It is just this characteristic which is utilized to entrap cells and tissues within hydrogels. The known cell microencapsulation technique consists of dropping an alginate and cell suspension into a bath containing calcium, and controlling the diameter of the microcapsules by various physical methods. The instantaneous formation of the gel allows cells to become entrapped within it. In the case of chondrocyte microencapsulation for repairing articular cartilage, the limitation on the use of alginate is due mainly to the absence of replication activity of the cells once inside. More recently another polysaccharide, basic in this case, has sparked a certain amount of interest in its potential use in the field of microencapsulation of biological materials, it being biocompatible and available in large quantities like alginate. This is chitosan. Chitosan is a basic polysaccharide of molecular weight between 50 and 1,500 kDa, consisting of a chain of D-glucosamine (GlcNH₂) residues interspersed with N-acetyl-glucosamine units, all joined by β1→4 bonds. It is normally insoluble in neutral or basic aqueous solutions; in acid solutions with pH ≤ 5, the free amino group is protonated to render the polymer soluble. This polymer is already widely used in the medical field in that it demonstrates a low immunological, pathological and infectious response (Suh Francis J.K., Matthew H.W.T. Biomaterials, 2000, 21, 2589-2598*;* Miyazaki S. et al. Chem. Pharm. Bull., 1981, 29, 3067-3069). Chitosan has all the ideal characteristics for use as a biomaterial due to its physico-chemical properties, such as high density of cationic charge in solution, and its high processability by virtue of which porous structures can be obtained into which cells can be implanted. Most of the recent research is focused on providing methods for enhancing the biological effects of chitosan. In particular most of the efforts have been aimed at increasing the polymer cationicity or modifying its bioavailability characteristics through (bio)chemical modifications. It is precisely in its derivatised forms that chitosan takes on those properties required for its use as a biomaterial. Recent studies have indeed shown that a particular derivative of chitosan with lactose is biocompatible and able to induce aggregation of chondrocytes in primary culture as well as stimulating therein the formation of characteristic markers of cartilaginous tissue, such as collagen type II and aggrecan (Donati, I. et al., Biomaterials 26, 2005, 987-998).

Modification of chitosan with saccharide side groups, for example by inserting lactose units through a reductive amination reaction, is known and leads to greater solubility in water of the polysaccharide derivatives as reported in US Patent 4,424,346 (Hall, L.D. and Yalpani, M.). US 4,424,346 also mentions that the lactose derivative of chitosan gives rigid gels in aqueous solutions at concentrations greater than 3-5%, whereas it neither gels nor precipitates in mixture with salts or acids (particularly of Ca, Cr, Zn chlorides, K chromate, boric acid) and combinations thereof. Again, the cited patent mentions that chitosan derivatised with another oligosaccharide, namely cellobiose, does not in itself form gels in aqueous solutions, but forms rigid gels when mixed with alginate. This gel formation is due to the strong interaction between the positive charges of the polycation and the negative charges of the polyanion which leads to system coacervation, a process which limits microencapsulation.

With the aim of obtaining three-dimensional polymer matrices suitable for incorporating biological materials or biologically active compounds, these matrices should preferably have good solubility in aqueous solution, such matrices needing in any event to have a certain degree of dispersibility in aqueous solution without giving rise to insoluble precipitates, hence ensuring a three-dimensional structure suitable for microencapsulation.

To overcome this, various systems are described which comprise the use of a mixture of polysaccharides, also modified and/or crosslinked, to improve the physico-chemical characteristics of these matrices.

In particular WO94/25080 (Griffith-Cima, L. et al.) describes injectable polysaccharide-cell compositions where the use of alginate in combination with other polysaccharides, essentially hyaluronic acid, is provided to obtain hydrogels suitable for encapsulating cells for tissue engineering. Polymers suited to the purpose of forming hydrogels for implanting isolated cells are most various and are crosslinked with crosslinking agents consisting of ions (preferably divalent or trivalent), and changes in pH and temperature. The ionic concentration for crosslinking is not less than 0.005 M. The hydrogels can also be complexed and stabilized with polycations selected from synthetic polyamines, such as polyethyleneamine, polyvinylamine, polyallylamine, and polysine.

Similarly, patent application WO96/40304 (Hubbell, J) describes hydrogels formed of polymers crosslinked with crosslinking agents consisting of ions, pH and temperature changes, radical initiators and enzymes. Polymers referred to include polysaccharides and these latter can be selected from modified alginate, modified hyaluronic acid, gellan and carrageenan.

US Patent 6,224,893 (Langer, R.S. et al.) describes interpenetrating polymer networks (IPNs) or semi-interpenetrating polymer networks for drug delivery and tissue engineering. Said IPNs consist of solutions in the form of preferably hydrophilic, ionically or covalently crosslinked polymer hydrogels, the ionically crosslinked polymers including: hyaluronic acid, dextran, heparan sulfate, chondroitin sulfate, heparin, alginate, gellan and carrageenan, while the covalently crosslinked polymers include chitosan crosslinked with isothiocyanate. IPNs are formed from two polymer components that are crosslinked, but not mutually, while the semi IPNs comprise two components of which only one is crosslinked, but never mutually. Preferentially, but not exclusively, polymer crosslinking is achieved by photoactivation of a radical photoinitiator. In this respect, polymer compositions can be formed from covalently crosslinked polymers by means of a photoinitiator, or mixtures of covalently and ionically crosslinkable polymers or hydrophilic polymers which form semi-IPNs when exposed to radiation.

US Patent 5,620,706 (Severian, D. et al., reports ionic complexes between acid and basic polysaccharides, and in particular coacervation of chitosan and xanthan, a polysaccharide that bears side chain negative charges, a characteristic which is make use of to obtain insoluble hydrogels.

WO 2005/061611 (White, B.J. et al.) describes the preparation of interpenetrating or semi-interpenetrating polymer networks consisting of a composition comprising crosslinked water soluble derivatives of a basic polysaccharide and a non-crosslinked anionic polysaccharide. In particular, said IPNs are obtained by mixing hyaluronic acid with crosslinked N-carboxymethyl, O-carboxymethyl, O-hydroxyethyl chitosan derivatives or with partially acetylated chitosans. These chitosan derivatives can be mixed in solution with hyaluronic acid as, according to the Inventors, they are solubilized under pH conditions such as not to have any positive charges on the chain and to avoid formation of ionic complexes. In this manner coacervation with the polyanion is prevented by the complete removal or compensation of the charge on one of the polymers. These are therefore solutions of polyanion/ neutral polysaccharide or polyanion/polyampholyte.

Despite this, the problem of providing biocompatible systems able to incorporate cells and maintain cell phenotype while at the same time allowing growth and replication is not yet resolved, as already previously discussed.

A first purpose of the present invention is therefore to establish biocompatible systems for tissue engineering as cell carriers, able not only to ensure their survival but also maintenance of their phenotypical characteristics, growth and replication.

A further purpose is to provide said systems by the use of easily commercially available polysaccharides, without said polysaccharides having been subjected to chemical manipulations, and without there being necessary complex manipulations in preparing said systems.

A further purpose is the provision of biocompatible systems in the form of hydrogels or 3D matrices, as cell carriers, that are easily usable in accordance with the various usage requirements and without further manipulations by the health technician.

A further purpose of the present invention is the provision of biocompatible systems in the form of hydrogels or 3D matrices, as drug carriers, that are easily usable in accordance with the various usage requirements.

### Summary of the invention

To pursue the aforementioned aims, the Inventors have identified suitable derivatives of basic polysaccharides which, when physically mixed with anionic polysaccharides, provide, under suitable conditions, aqueous solutions of said polysaccharides without generating insoluble coacervates.

The aqueous solutions of the mixtures can subsequently be gelled with suitable gelling agents, to obtain three-dimensional hydrogels or matrices in which the cells or drugs to be carried can be incorporated. The polysaccharide mixtures for preparing the 3D matrices of the invention comprise anionic polysaccharides and oligosaccharide derivatives of chitosan.

Surprisingly, in addition to giving rise to compositions in which said physical mixtures of polyanionic polysaccharides and polycationic polysaccharide derivatives are soluble in aqueous environment, said compositions when treated with suitable gelling agents give rise to hydrogels able to microencapsulate cells and in which said cells maintain their phenotype and are able to proliferate. Although alginate presents many advantages for this use, the technique of microencapsulation can effectively be carried out with all ionic polysaccharides that form hydrogels instantaneously on contact with solutions of ions (lyotropic) or with cooled solutions (thermotropic). The initially stated class of polysaccharides includes, for example, pectate and carrageenan as well as alginate. The second class of polysaccharides includes for example agarose (partially sulphated), gellan as well as carrageenan again.

A first aspect of the invention is therefore compositions consisting of hydrogels **characterised in that** they are obtainable from aqueous solutions of mixtures of at least one lyotropic or thermotropic anionic polysaccharide and at least one derivative of chitosan with oligosaccharides, wherein said chitosan derivatives have a degree of derivatization of at least 40% and wherein said aqueous solutions have an ionic strength of at least 50 mM and not greater than 175 mM and a pH of at least 7, by means of treating said aqueous solutions of polysaccharide mixtures with agents capable of gelling the lyotropic or thermotropic polyanionic polysaccharides comprised within the mixtures themselves.

Another aspect of the invention is a process for preparing said compositions.

A further aspect of the invention is the use of said compositions for cell microencapsulation for use in tissue engineering or microencapsulation of active compounds for their use in biomedicine.

### Brief description of the figures

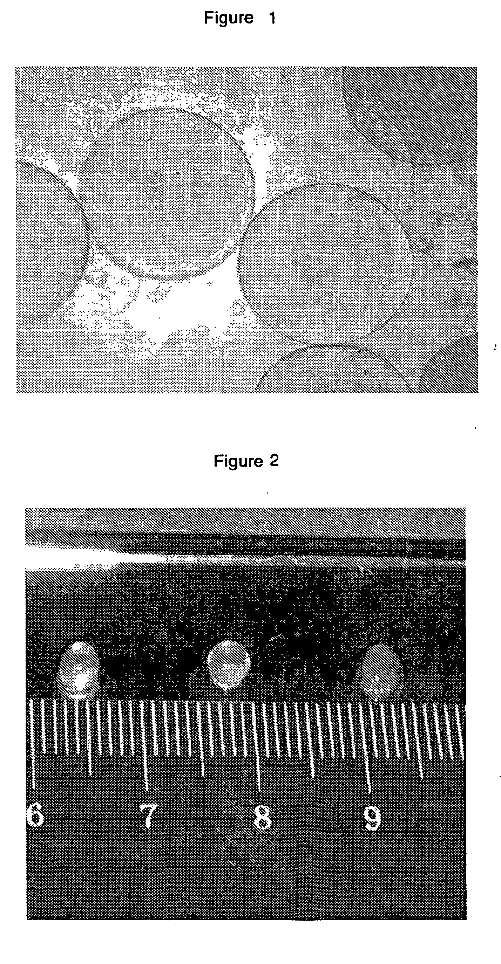

Figure 1: Optical microscope photograph of microcapsules obtained according to example 6 from binary polymer solutions of alginate and lactose derivative of chitosan (known hereinafter indicated as chitlac) prepared in NaCl 0.15M, Hepes 10 mM, pH 7.4. Total polymer concentration 2%, weight ratio of polyanion to polycation 3:1. Microcapsules obtained by dropping the binary solution into a solution containing CaCl₂ 50 mM, mannitol 0.15M, Hepes 10 mM, pH 7.4. Conditions for electrostatic bead generator: voltage 5kV, internal diameter of needle 0.7 mm, distance between gelling bath and needle 4 cm, flow rate of binary polymer solution 10 mL/min. Mean capsule diameter 870 ± 20 µm.
Figure 2: Microcapsules obtained by means of syringe with 23G needle, starting from binary polysaccharide solution of A) alginate and chitlac dropped into a gelling bath containing CaCl₂ 50 mM (ex. 2); B) κ-carrageenan and chitlac dropped into a gelling bath containing KCI 100 mM (ex. 4); C) agarose (partially sulphated) and chitlac dropped into a gelling bath formed of water cooled to about 30°C (ex. 5). In all the stated cases, total polysaccharide concentration is 3% and the weight ratio of polyanion to chitlac is 1:1.

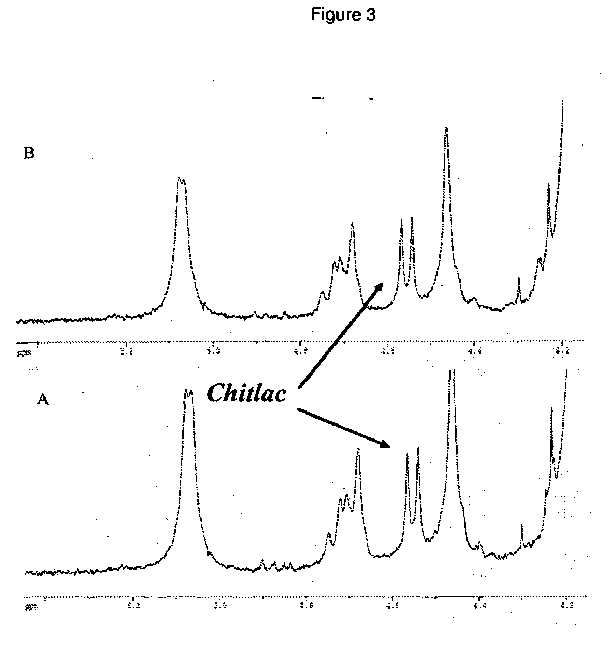

Figure 3: Protonic ¹H-NMR analysis of the binary mixture of alginate (1.5%) and chitlac (0.5%) undertaken before (A) and after (B) microcapsule formation, in accordance with example 6, from which it can be seen that chitlac is present in both cases.

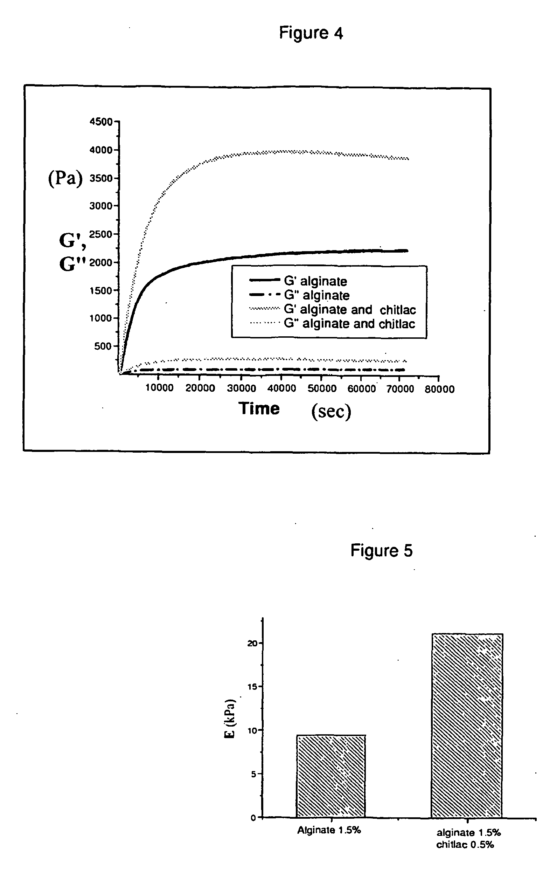

Figure 4: Time variation of the elastic modulus (G' = ■) and viscous modulus (G" = □) for an alginate solution (polymer concentration 1.5%, NaCl 0.15 M, Hepes 10 mM, pH 7.4) and for a binary solution of alginate and chitlac (G' = • ; G" = ○) (total polymer concentration 2%, weight ratio of alginate to chitlac 3:1, NaCl 0.15 M, Hepes 10 mM, pH 7.4). Gelling achieved by means of the *in-situ* technique with CaCO₃ 15 mM and GDL 30 mM (ex. 9).
Figure 5: Compression modulus (E) measured on cylindrical hydrogels obtained by means of the *in-situ* technique, starting from an alginate solution (1.5% concentration) and from a binary solution of alginate and chitlac, total polymer concentration 2%, weight ratio of alginate to chitlac 3:1) (ex. 9). In both solutions NaCl 0.15 M, Hepes 10 mM, pH 7.4 were used.

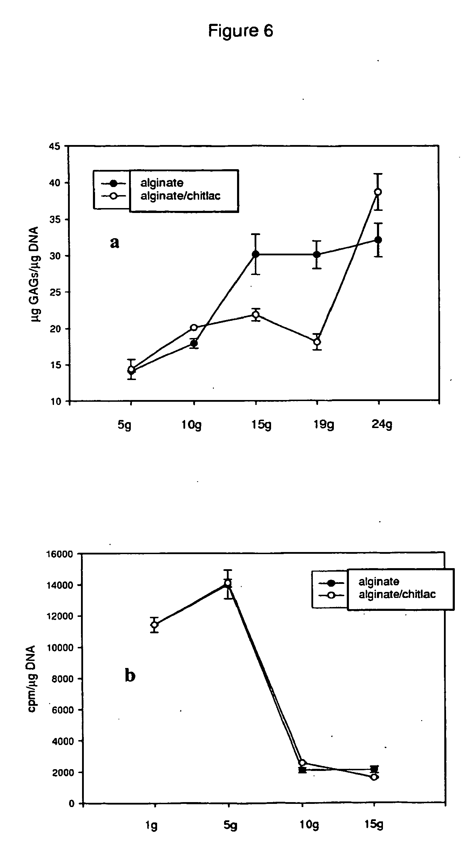

Figure 6: A) proteoglycan content measured by colorimetry (dimethylmethylene blue) of chondrocyte culture maintained in capsules of alginate and alginate chitlac (prepared as in ex. 12). B) assessment of level of collagen synthesis by means of assay of incorporated [³H-proline] in chondrocyte culture maintained in capsules of alginate and alginate chitlac (prepared as in ex. 12).

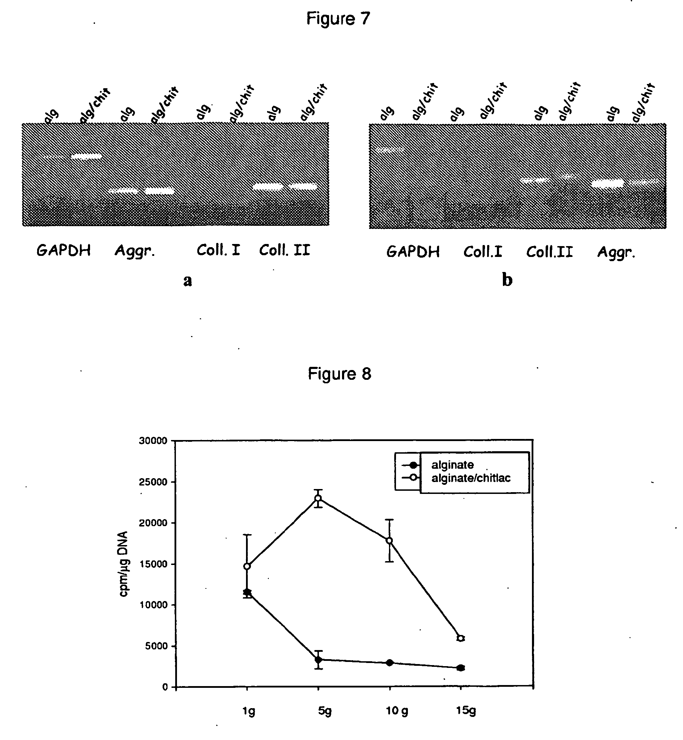

Figure 7: RT-PCR analysis for assessment of collagen I, collagen II and aggrecan expression in chondrocytes grown in alginate capsules and in alginate:chitlac capsules prepared as in ex. 12. In figure A extraction of RNA from chondrocytes was undertaken 2 days after encapsulation, and in figure B, 17 days after encapsulation.
Figure 8: Proliferation assay with [³H-thymidine]. The upper curve shows the results obtained with cells in alginate/chitlac capsules prepared as in ex. 12, which show an elevated proliferation activity up to the fifteenth day of culture. The lower curve shows the results obtained with cells in alginate capsules with poor replication capacity. The experimental data clearly and unequivocally show that after the very first days of culture, chondrocytes in the alginate capsules block replication, whereas a rapid cell replication is observed in the mixed capsules, which extends up to the first two weeks of culture.

### Detailed description of the invention

### Definitions

The terms "hydrogel" or "hydrogels" indicate highly hydrated semi-solid structures able to maintain form and dimension when not subjected to deformation. Hydrogels can be obtained from concentrated solutions of suitably crosslinked polysaccharides.

The terms "3D matrices" or "three-dimensional matrices" indicate solid or semi-solid structures able to maintain form and dimension when not subjected to deformation. 3D matrices can be obtained from concentrated solutions of suitably crosslinked polysaccharides.

Accordingly, the terms "hydrogels" or "3D matrices" are to be considered the same for the purposes of the detailed description of the invention that follows.

The term "microencapsulation" indicates the process of inclusion of materials, be they biological or otherwise, inside the hydrogels usually, but not exclusively, in spherical form of millimetric or micrometric size formed from lyotropic or thermotropic polysaccharides following treatment with suitable ions in the first case (i.e. lyotropic polysaccharides) and with cooled solutions in the second case (i.e. thermotropic polysaccharides).

### Description

The aims and advantages of the three-dimensional matrices obtainable from at least binary solutions of gelled polysaccharides of the present invention, will be better understood during the detailed description that follows where, for the purposes of non-limiting illustration, some examples of hydrogels and their physico-chemical characterization will be described together with their biological compatibility/biological properties with isolated cells encapsulated therein.

For the pursued aims, some thought was given to the identification and planning of a biomaterial for cell encapsulation possessing those characteristics, in terms of physiological markers, which are most similar to those of the extracellular matrix for developing a more effective method of culturing cells and in particular chondrocytes. In particular biopolymers already widely employed in tissue engineering were used, i.e. acidic polysaccharides such as alginate and chitosan modified with oligosaccharides as aforesaid. The combination of an anionic polysaccharide and a basic polysaccharide can however lead, as previously noted, to coacervation of the system with consequent loss of the three-dimensional structure suitable for inclusion of biological material. The formation of coacervates thus constitutes in every respect a drawback for the microencapsulation of biological materials or biologically active molecules. Indeed for microencapsulation, the use of soluble formulations of starting polysaccharides is of great importance, as the entrapment of biological material such as cells or compounds of varying nature within hydrogels is possible, as known to an expert of the art, by adding a polymer solution drop-wise into a solution containing suitable crosslinking ions. If coacervates are present or are formed in the polymer solution, on coming into contact with the solution of crosslinking ions they lead to the formation of fibrous precipitates which are unable to include the biological material itself. Hence the importance of identifying the solubility window for the binary polysaccharide mixtures which, suitably treated with gelling agents, be they ion-containing solutions or cooled solutions, lead to formation of the hydrogel. Consequently, in pursuing the aims of the invention and contrary to that reported in US Patent 4,424,346, the composition of polysaccharides required to obtain the 3D matrices of the invention comprises at least binary mixtures of an anionic polysaccharide and an oligosaccharide derivative of chitosan characterized by being, when under suitable conditions, soluble in aqueous solutions and by not generating insoluble coacervates. In this respect, the Inventors have surprisingly found that mixtures comprising at least one anionic polysaccharide and at least one oligosaccharide derivative of chitosan in aqueous solutions, having said chitosan derivatives a derivatisation of at least 40% and having said aqueous solutions a pH within the physiological range, in particular from 7 to 8, and a suitable ionic strength, in particular of at least 50 mM and not greater than 175 mM, do not result in coacervation of the two polysaccharides which hence remain in solution. In these conditions the anionic and cationic polysaccharides do not originate in an- aqueous environment coacervates or precipitates until a total polymer concentration up to 3%.

The objects of the present invention in all its preferred aspects and derivations arise from this observation as described in the pending Italian patent application -no. PD2006A000202.

Formation of the three-dimensional hydrogel or matrix is obtainable from said binary solution by treating it with suitable gelling agents capable of gelling the polyanionic polysaccharide. In this respect, the invention provides compositions consisting of a hydrogel obtainable from aqueous solutions of mixtures of at least one lyotropic or thermotropic anionic polysaccharide and at least one oligosaccharide derivative of chitosan, in which said chitosan derivatives have a degree of derivatization of at least 40 % and in which said aqueous solutions have an ionic strength of at least 50 mM and not greater than 175 mM and a pH of at least 7, by means of gelification with gelling agents of the lyotropic or thermotropic anionic polysaccharides comprised in the mixtures themselves. By this gelling process of the polyanionic polysaccharide the chitosan derivative is entrapped in the hydrogel. Hence the hydrogel resulting from the gelification of aqueous solutions of mixtures of at least one lyotropic or thermotropic anionic polysaccharide and at least one oligosaccharide derivative of chitosan, in which said chitosan derivatives have a degree of derivatization of at least 40 % and in which said aqueous solutions have an ionic strength of at least 50 mM and not greater than 175 mM and a pH of at least 7, are formed by the reticulated lyotropic or thermotropic anionic polysaccharide entrapping the chitosan derivative.

For preparing the hydrogels of the present invention, chitosan can be derivatized with oligosaccharides comprising from 1 to 4 glycoside units and in a preferred aspect said oligosaccharides comprise from 2 to 4 glycoside units and more preferably are selected from the group consisting of lactose, cellobiose, cellotriose, maltose, maltotriose, maltotetraose, chitobiose, chitotriose, melibiose. The average molecular weight (known hereinafter as MW) of chitosan usable for obtaining said oligosaccharide derivatives can reach 1,500 kDa and can preferably be within the range from 400 kDa to 1,000 kDa. In addition, for the purposes of the present invention, the degree of substitution of the chitosan amine groups with said oligosaccharides has to be above 40-45% (∼ 45%). Preferably, the degree of substitution of the chitosan amine groups with oligosaccharides is within the range comprised from 50% to 80% and is more preferably 70%.

The preparation process of said oligosaccharide derivatives of chitosan is a known process and comprises treating a solution of chitosan in acetic acid (pH 4.5) and methanol with a reducing sugar, such as lactose, in the presence of sodium cyanoborohydride. The interaction between the chitosan amine groups and the lactose aldehyde group leads to the formation of an unstable intermediate known as a Schiff base. This is reduced in the presence of borohydride leading to the formation of a stable secondary amine.

Regarding the polyanionic polysaccharides, the hydrogels of the present invention can be obtained with polysaccharide mixtures comprising lyotropic anionic polysaccharides, and in this case in a preferred aspect these are selected from the group consisting of carrageenans, pectates and pectinates, alginates, gellan, rhamsan, welan, xanthan, or thermotropic anionic polysaccharides in which case they are preferably selected from the group consisting of partially sulphated agarose, carrageenan, cellulose sulphate, gellan, rhamsan, welan, xanthan. The polysaccharides gellan, rhamsan, welan, xanthan have, as known, a chemico-physical behaviour both lyotropic and thermotropic, then gelling agents usable can be chemical agents such as ions and physical agents such as temperatures. The average molecular weight (MW) of the polyanions can be up to 2,000 kDa and can preferably be from 100 kDa to 1,000 kDa and more preferably are used at average molecular weights of 200 kDa.

In another preferred aspect weight ratios between the polymers of the polysaccharide mixture is from 1 to 1 to 3 to 1 (polyanion:chitosan derivative).

For the purposes of the present invention the binary mixtures of the chitosan-derivative and the polyanion can be up to a total polymer concentrations of 3% w/v (g/mL). -Preferably said total polymer concentrations are within the range comprised from 1.5% w/v (g/mL) to 3% w/v (g/mL) and more preferably.can be 2% w/v (g/mL).

The at least binary solutions of polysaccharides necessary for preparing the hydrogels of the present invention have a pH within the physiological range, and in particular between 7 and 8, being more preferably pH 7.4, and have an osmolarity between 250 and 300 mM with an ionic strength between 50 mM and 175 mM obtainable preferably by addition of NaCl in concentrations between 0.05 M and 0.15 M, being more preferably 0.15 M. The osmolarity is preferably obtained with non-ionic solutes such as mannitol.

The gelling agents can be chosen, according to the type of lyotropic anionic polysaccharide, from suitable monovalent, divalent or trivalent ions and for thermotropic polysaccharides, from temperatures not higher than 40°C or not lower than 10°C.

In the case of binary polysaccharide solutions containing a chitosan derivative and lyotropic polyanions, hydrogels are obtained by treating the aforesaid with suitable alkaline or alkaline earth ions or transition metals or rare earth metals at suitable concentrations according to the anionic polysaccharide.

Preferably when the gelling agents are chosen from monovalent ions, these are selected from the group consisting of potassium, rubidium, caesium, thallium, silver and mixtures thereof, whereas when they are divalent ions, these are selected from the group consisting of Ca²⁺, Ba²⁺, Sr²⁺, Cu²⁺, Pb²⁺, Mg²⁺, Mn²⁺, Zn²⁺ and mixtures thereof, whereas when they are selected from trivalent ions, these are from the group consisting of Al³⁺, Fe³⁺, Gd³⁺, Tb³⁺, Eu³⁺ and mixtures thereof.

For polysaccharides such as alginate and pectate, said ions are alkaline earth ions, excluding magnesium, and transition metals, being in this case preferably selected from the group consisting of calcium, barium, strontium, lead, copper, manganese and mixtures thereof or rare earth ions, being preferably elected from the group consisting of gadolinium, therbium, europium and mixtures thereof. The concentrations of the aqueous solutions of said suitable ions for gelling binary polysaccharide solution are higher than 10 mM, preferably between 10 mM and 100 mM and more preferably 50 mM. In addition the gelling solution can contain ionic osmolites (such as NaCl) or non-ionic osmolites (such as mannitol) to obtain a gelling solution with an osmolarity up to 0.3 M. Preferably the gelling solution contains a CaCl₂ concentration of 50 mM and concentration of NaCl of 0.075 M, or a concentration of non-ionic osmolites (such as mannitol) of 0.15 M.

In the case of carrageenans, alkaline ions are used and preferably selected from the group consisting of potassium, rubidium and caesium, at concentrations not less than 50 .mM, being preferably between 50 mM and 100 mM and more preferably 0.1 M.

In the case of polysaccharide solutions containing a chitosan derivative and polyanions that give thermotropic hydrogels, such as partially sulphated agarose, the preparation of hydrogels is carried out by cooling to a temperature lower than that of gel formation. In particular, for thermotropic polysaccharides, when the gelling agent is temperature, this latter is preferably within the range from 40°C to 10°C.

The polysaccharide solutions are prepared at a temperature above that of hydrogel formation by the thermotropic polysaccharide. At this temperature the thermotropic polysaccharide does not form hydrogels. Preferably the temperature at which the polysaccharide solutions are prepared is within the range from 50°C to 30°C, being more preferably 37°C. Hydrogel formation occurs by dropping the polysaccharide mixture solution into a gelling bath and cooling the same to a temperature lower than that of gel formation. Preferably, this temperature is within the range from 10°C to 40°C, being more preferably 20°C.

In a preferred aspect the hydrogels of the invention are prepared starting from polyanion-polycation binary polysaccharide mixtures, where the polyanion is represented preferably by alginate and the polycation by oligosaccharide derivatives of chitosan, being preferably the lactose derivative of chitosan (chitlac). For the purposes of the present invention, the biological material or active compounds to be carried are added to the aqueous polymer solutions before hydrogel preparation by treatment with suitable gelling agents.

The 3D matrices or hydrogels of the invention are obtainable in accordance with known methods and in particular comprise at least the following steps:
a) preparing an aqueous solution of a mixture of at least one lyotropic or thermotropic anionic polysaccharide and at least one derivative of chitosan with oligosaccharides, said chitosan derivatives having a degree of derivatization of at least 40% and said aqueous solutions having an ionic strength of at least 50 mM and not greater than 175 mM and a pH of at least 7, then optionally adding cells and/or active compounds to the prepared polysaccharide solutions ;
b) adding the solution prepared in step a) by a suitable means for obtaining the required hydrogel form, such as for example dropping by means of a needle, to a gelling solution either containing the crosslinking ion for lyotropic anionic polysaccharides or being at a suitable temperature for the thermotropic polyanions;
c) removing the formed hydrogel, optionally incorporating the cells and/or the active compounds, by suitable methods such as for example centrifugation or dialysis.

In the case of dropping, the drop size, controllable by various physical methods (e.g. choice of external diameter of needle, presence of an electric field or an air flow coaxial to the needle), determines the final hydrogel size in microcapsule form. The capsules are left for example in the gelling solution for about 10 minutes and then removed.

With the afore-described methods, hydrogels can be obtained which, with appropriate and further treatment, can assume various forms, preferably microcapsules, but also cylinders or discs.

In particular, the steps leading to formation of hydrogel cylinders starting from binary polymer solutions are the following: a) the binary polymer solution to which the cells and/or the active compounds to be encapsulated are added, is transferred into cylindrical or discoidal containers and closed at the ends with dialysis membranes; b) these containers are then immersed into the solution either containing the crosslinking ion or being at a suitable temperature (gelling solution). The cylindrical or discoidal containers are left in the gelling solution for about 30 minutes and then removed; c) the gel cylinders or discs of hydrogels obtained are taken from the containers after removing the dialysis membranes.

Alternatively, in the case of the alginate, the cylinders can be prepared by adding an inactive form of the crosslinking ion, e.g. CaCO₃ or the Ca-EDTA complex, to the polysaccharide solution. A substance which slowly hydrolyzes the Ca salts, such as GDL (D-glucono-δ-lactone) is then added. This suspension is transferred into the cylindrical or discoidal containers and maintained therein for 24 hours. The gel cylinders or discs of hydrogels are then taken from the containers. This methodology is described as cylinder formation by *in situ* calcium release.

By way of non-limiting illustration, described in the following is the general preparation of the hydrogels or 3D matrices in the form of microcapsules and cylinders in accordance with the invention obtained from the binary polysaccharide solutions of at least one lyotropic or thermotropic anionic polysaccharide and at least one oligosaccharide derivative of chitosan and examples of said microcapsule preparation with incorporated isolated cells.

### A. Preparation of 3D matrices from a solution of an anionic polysaccharide and an oligosaccharide derivative of chitosan.

### Microcapsule preparation

An aqueous solution of a binary mixture of lyotropic or thermotropic anionic polysaccharides is prepared for example as following:
a- a solution of an anionic polysaccharide, for example alginate (M_{w} ∼ 130,000), having an ionic strength of 150 mM by addition NaCl 0.15 M, Hepes 10 mM at pH 7.4 is prepared;
b- a solution of a chitosan derivative, for example chitlac (M_{w} ∼ 1.5x10⁶), having the same ionic strength of 150 mM by addition of NaCl 0.15 M, Hepes 10 mM at pH 7.4 is prepared;
c- the two solutions are mixed by a magnetic stirrer to obtain a solution containing alginate and chitlac (for example in a weight ratio 1:1, having a total polymer concentration of 2%).

The aqueous solutions so prepared are completely transparent and without precipitates and/or coacervates.

The same procedure can be applied with other lyotropic or thermotropic polysaccharides and chitosan derivatives as well as with the different w/w ratios anionic polysaccharide:chitosan derivative before mentioned and different total polymer concentrations before mentioned without formation of precipitates and/or coacervates.

Hence, the microcapsules of the particular examples given below were prepared in accordance with known methods and in particular: a) by the use of simple syringes with which the binary aqueous solutions of an anionic polysaccharide and an oligosaccharide derivative of chitosan are dropped into a suitable gelling bath; b) using an Electronic Bead Generator, developed by Prof. Gudmund Skjåk-Bræk of the Institute of Biotechnology of NTNU University of Trondheim (Norway) described by Strand et al., 2002, J. of Microencapsulation 19, 615-630*.* Said system consists of an electrostatic generator with a voltage (up to 10 kV) adjustable by means of a suitable switch, connected to a support for an autoclavable needle contained in a plexiglass safety cage. By means of a system external to the cage, consisting of a syringe regulated by a pump and connected to a latex tube of internal diameter 1 mm, an alginate solution is dropped onto a crystallizer (inside the cage) containing the gelling solution into which an electrode is inserted. The instrument generates a constant potential difference between the point of the needle and the free surface of the gelling solution which can be regulated and which varies from 0 to 10 kV. The difference in potential causes the sudden detachment of the polymer droplet (negatively charged) from the point of the needle hence enabling a capsule, even of very small size (<200 µm) to be obtained. Capsule size can also be regulated by varying other factors, such as the internal diameter of the needle, the distance between the needle and the surface of the gelling solution, flow rate of the polymer.

### Preparation of cylinders

The gel cylinders and discs of the particular examples given below were prepared by pouring the binary aqueous polysaccharide solution into cylindrical or discoidal containers. The dimensions of the cylindrical or discoidal hydrogels depend on the container dimensions. Typically the dimensions of the cylindrical containers are 18 mm in height and 16 mm in internal diameter, although different dimensions (height and internal diameter) are possible.

Examples of hydrogel preparation starting from binary polysaccharide mixtures of chitosans modified with oligosaccharides and lyotropic or thermotropic polyanions polysaccharides are given hereinafter.

To prepare the 3D matrices in accordance with the following examples, aqueous solutions of commercial anionic polysaccharides and of the lactose derivative of chitosan prepared by reductive amination as described hereafter in example 1 were used.

### Example 1: Synthesis of the chitosan derivative containing lactose (chitlac)

Chitosan (1.5 g, degree of acetylation 11 %) is dissolved in 110 mL of a solution of methanol (55 mL) and a 1% acetic acid buffer at pH 4.5 (55 mL). Added to this are 60 mL of a solution of methanol (30 mL) and 1% acetic acid buffer at pH 4.5 (30 mL) containing lactose (2.2 g) and sodium cyanoborohydride (900 mg). The mixture is left under agitation for 24 hours, transferred into dialysis tubes (cut off: 12,000 Da) and dialyzed against 0.1 M NaCl (2 changes) and against deionised water until the conductivity is 4 µS at 4°C. Finally, the solution is filtered through 0.45 µm Millipore filters and lyophilized.

### Example 2: Preparation of microcapsules from 3:1 w/w alginate:chitlac 2% w/v (g/mL) binary polysaccharide solution, by means of a syringe

A binary polysaccharide solution containing chitlac (ex.1, MW ∼ 1,500 kDA) and alginate (MW ∼ 130 kDa) (total polymer concentration 2%, ratio of alginate to chitlac = 3:1) containing NaCl 0.15 M, Hepes 10 mM, pH 7.4 was prepared. 20 mL of the solution was added drop-wise, using a syringe equipped with a 23G needle, to a solution containing 50 mM CaCl₂ and 0.15 M mannitol (gelling bath) under agitation by magnetic stirrer. The capsules were maintained under agitation in the gelling bath for 10 minutes before being removed and washed with deionised water.

### Example 3: Preparation of microcapsules from 3:1 w/w alginate:chitlac 2% w/v (g/mL) binary polysaccharide solution, by means of a syringe

A binary polysaccharide solution containing chitlac (ex.1, MW ∼ 1,500 kDA) and alginate (MW ∼ 130 kDa) (total polymer concentration 2%, ratio of alginate to chitlac = 3:1) containing NaCl 0.15 M, Hepes 10 mM, pH 7.4 was prepared. 20 mL of the solution was added drop-wise, using a syringe equipped with a 23G needle, to a solution containing 50 mM CaCl₂ and 0.075 M NaCl (gelling bath) under agitation with magnetic stirrer. The capsules were maintained under agitation in the gelling bath for 10 minutes before being removed and washed with deionised water.

### Example 4: Preparation of microcapsules from 3:1 w/w carrageenan:chitlac 2% w/v (g/mL) binary polysaccharide solution, by means of a syringe

A binary polysaccharide solution containing chitlac (ex.1, MW ∼ 1,500 kDA) and carrageenan (MW ∼ 300 kDa) (total polymer concentration 2%, ratio of carrageenan to chitlac = 3:1) containing NaCl 0.15 M, Hepes 10 mM, pH 7.4 was prepared. 20 mL of the solution was added drop-wise, using a syringe equipped with a 23G needle, to a solution containing 100 mM KCI (gelling bath) under agitation with magnetic stirrer. The capsules were maintained under agitation in the gelling bath for 10 minutes before being removed and washed with deionised water.

### Example 5: Preparation of microcapsules from 3:1 w/w partially sulfated agarose:chitlac 2% w/v (g/mL) binary polysaccharide solutions, by means of a syringe

A binary polysaccharide solution containing chitlac (ex.1, MW ∼ 1,500 kDA) and partially sulfated agarose (low gelling point) (total polymer concentration 2%, ratio of (partially sulfated) agarose to chitlac = 3:1) containing NaCl 0.15 M, Hepes 10 mM, pH 7.4 at about 60°C was prepared. 20 mL of the solution, cooled to about 30°C, was added drop-wise using a syringe equipped with a 23G needle, to a solution containing deionised water at about 4°C. The capsules were maintained under agitation in the cooled solution for 10 minutes before being removed and washed with deionised water.

### Example 6: Preparation of microcapsules from 3:1 w/w alginate:chitlac 2% w/v (g/mL) binary polysaccharide solution, by means of an Electrostatic Bead Generator

A binary polysaccharide solution containing chitlac and alginate (total polymer concentration 2%, ratio of alginate to chitlac = 3:1) containing NaCl 0.15 M, Hepes 10 mM, pH 7.4 was prepared. 20 mL of the solution was added drop-wise to a solution containing CaCl₂ 50 mM and mannitol 0.15 M (gelling bath). Drop dimensions were controlled by using an Electrostatic Bead Generator. Typically the usage conditions were: voltage 5kV, internal needle diameter 0.7 mm, distance between gelling bath and needle 4 cm, flow rate of binary polymer solution 10 mL/min. The capsules were left in the gelling solution under agitation for 10 minutes before being removed and washed with deionised water.

### Example 7: Preparation of microcapsules from 3:1 w/w alginate:chitlac 2% w/v (g/mL) binary polysaccharide solution, by means of an Electrostatic Bead Generator

A binary polysaccharide solution containing chitlac and alginate (total polymer concentration 2%, ratio of alginate to chitlac = 3:1) containing NaCl 0.15 M, Hepes 10 mM, pH 7.4 was prepared. 20 mL of the solution was added drop-wise to a solution containing CaCl₂ 50 mM and NaCl 0.075 M (gelling bath). Capsule size was controlled by using an Electrostatic Bead Generator as described in the preceding example. The capsules were left in the gelling solution under agitation for 10 minutes before being removed and washed with deionised water.

### Example 8: Preparation of hydrogel cylinders starting from 3:1 w/w alginate:chitlac 2% w/v (g/mL) binary polysaccharide solution by means of dialysis

A binary polysaccharide solution containing chitlac and alginate (total polymer concentration 2%, ratio of alginate to chitlac = 3:1) containing NaCl 0.15 M, Hepes 10 mM, pH 7.4 was prepared. 20 mL of the solution was transferred into plastic cylinders 16 mm (Ø) x 18 mm (h) in size closed at the lower and upper ends with dialysis membranes (cutoff: ∼ 12,000). The cylinders containing the binary polysaccharide solution were immersed in 1 litre of a solution containing CaCl₂ 50 mM and NaCl 0.15 M for 30 minutes before being removed from the gelling solution.

### Example 9: Preparation of hydrogel cylinders starting from 3:1 w/w alginate:chitlac 2% w/v (g/mL) binary polysaccharide solution by means of in situ calcium release

A binary polysaccharide solution containing chitlac and alginate (total polymer concentration 2%, ratio of alginate to chitlac = 3:1) containing NaCl 0.15 M, Hepes 10 mM, pH 7.4 was prepared. CaCO₃ (final concentration 15 mM) and GDL (D-glucono-δ-lactone) (final concentration 30 mM) were added to 20 mL of the solution. The mixture was transferred into plastic cylinders 16 mm (0) x 18 mm (h) in size. The mixture was left at ambient temperature for 24 hours then the gels were removed.

### Example 10: Encapsulation of chondrocytes in microcapsules obtained starting from 3:1 w/w alginate:chitlac 2% w/v (g/mL) binary polysaccharide solution

Chondrocytes, extracted from articular cartilage of pig, were suspended at a density of 5 x 10⁵ cells/mL in a mixture of 1.5% alginate and 0.5% chitlac prepared in a buffer of 0.15 NaCl, 10 mM Hepes, pH 7.4. The cell suspension was gently stirred and dropped from an Electronic Bead Generator into a gelling solution composed of CaCl₂ 50 mM, mannitol 0.15 M, Hepes 10 mM, pH 7.4. The capsules were left to gel completely under light agitation for 10 minutes, then collected and cultured in DMEM medium (Dulbecco's Modified Eagle's Medium) and withdrawn at successive time intervals for undertaking the various biochemical assays.

### Example 11: Encapsulation of chondrocytes in microcapsules obtained starting from 3:1 w/w alginate:chitlac 2% w/v (g/mL) binary polysaccharide solution

Chondrocytes, extracted from articular cartilage of pig, were suspended at a density of 5 x 10⁵ cells/mL in a mixture of 1.5% alginate and 0.5% chitlac prepared in a buffer of 0.15 NaCl, 10 mM Hepes, pH 7.4. The cell suspension was gently stirred and dropped from an Electronic Bead Generator into a gelling solution composed of CaCl₂ 50 mM, mannitol 0.15 M, Hepes 10 mM, pH 7.4. The capsules were left to gel completely under light agitation for 10 minutes, then collected and cultured in DMEM medium.

### Example 12: Encapsulation of chondrocytes in capsules obtained starting from 3:1 w/w alginate:chitlac 2% w/v (g/mL) binary polysaccharide solution, by means of a syringe

Chondrocytes, extracted from articular cartilage of pig, were suspended at a density of 5 x 10⁵ cells/ml in a mixture of 1.5% alginate and 0.5% chitlac prepared in a buffer of 0.15 NaCl, 10 mM, Hepes, pH 7.4. The cell suspension was gently stirred and dropped from a 23G springe into a gelling solution composed of CaCl₂ 50 mM, mannitol 0.15 M, Hepes 10 mM, pH 7.4. The capsules were left to gel completely under light agitation for 10 minutes, then collected and cultured in DMEM medium.

### Example 13: Preparation of microcapsules of 3:1 w/w alginate-rhodamine:chitlac-fluorescein 2% w/v (g/mL) binary polysaccharide solution

A binary polysaccharide solution containing chitlac labelled with fluorescein and alginate labelled with rhodamine (total polymer concentration 2%, alginate to chitlac ratio = 3:1) containing NaCl 0.15 M, Hepes: 10 mM, pH 7.4 was prepared. 20 mL of the solution was added drop-wise to a solution containing CaCl₂ 50 mM and mannitol 0.15 M (gelling bath). Capsule size was controlled by the use of an Electrostatic Bead Generator. Typically the conditions used were: voltage 5 kV, internal diameter of needle 0.7 mm, distance between gelling bath and needle 4 cm, flow rate of binary polysaccharide solution 10 mL/min. The capsules were left in the gelling solution under agitation for 10 minutes before being removed and washed with deionised water.

### B. Characterization of hydrogels from a solution of an anionic polysaccharide and an oligosaccharide derivative of chitosan.

Capsule/cylinder size is clearly dependent on the preparation process used therefor. Figure 1 shows, by way of example, an optical microscope photograph of microcapsules prepared using an Electronic Bead Generator, while figure 2 shows larger sized capsules obtained starting from polyanion/polycation binary polysaccharide mixtures in aqueous solution by the use of simple syringes and adding the solution drop-wise into a suitable gelling bath. The cylinders were prepared in accordance with the aforegiven examples and are 16 mm (Ø) x 18 mm (h) in size.

The 3D matrix formation process occurs as soon as the binary polymer solution comes into contact with the gelling solution at appropriate ion content conditions for the lyotropic polyanions or with the cooled solution for the thermotropic ones. For example, formation of the hydrogels occurs instantly when the binary polymer solution containing the polysaccharides, in particular alginate and chitlac, comes into contact with the gelling solution containing an appropriate concentration of calcium ions. It follows that the modified chitosan, i.e. chitlac, while not forming part of the hydrogel structure, remains trapped within it. This has been confirmed by analysis with confocal microscope of capsules obtained from a binary polysaccharide mixture containing alginate labelled with rhodamine and chitlac labelled with fluorescein, prepared as described in example 13. The presence of both fluorophores inside the capsule indicated the presence of both polysaccharides in the final product. Furthermore, protonic NMR analysis undertaken on the binary polysaccharide solution before and after forming the microcapsules, prepared as in example 6, has clearly shown that chitlac is present in both cases (fig. 3).

Analysis of the mechanical properties of the hydrogels obtained from the binary polysaccharide solution containing polysaccharides, in particular chitlac and alginate, was undertaken on matrices of cylindrical form obtained from the *in situ* technique (example 9). A rheological study was carried out on formation kinetics of the gel and on its elastic properties. In particular, figure 4 shows the progression of the elastic modulus (G') and the viscous modulus (G") over time for the hydrogel obtained starting from the binary aqueous solution of chitlac and alginate and for that obtained starting from the aqueous solution of alginate alone.

It should be noted that the presence of modified chitosan, i.e. chitlac, does not alter formation kinetics of the hydrogel, seeing that the rate of increase of G' is comparable in both cases. However, it is interesting to note that the maximum value reached by G' is greater in the case of the hydrogel obtained starting from the binary solution of chitlac and alginate. This leads to the conclusion that the hydrogels obtainable from the polymer mixtures exhibit better mechanical properties than those obtainable from solutions containing only the gelling polymer (for example, alginate).

This is confirmed by comparing these results with the compression modulus value measured on the hydrogel cylinders obtained, again from the *in situ* formation technique (example 9), from binary solutions of chitlac and alginate and from solutions of alginate alone. In a manner similar to that occurring with G', the compression modulus of hydrogels obtained from the aqueous solution of binary mixture of chitlac and alginate is greater than that of the gel obtained from the solution containing simply alginate (fig. 5).

### C. Biological tests on capsules of alginate (1.5%)/chitlac (0.5%) with chondrocytes

The microcapsules obtained as in example 12, cultured in DMEM medium (Dulbecco's Modified Eagle's Medium), were withdrawn at successive time intervals to measure their viability, using a cytotoxicity kit (live/dead cytotoxicity kit), based on the difference in permeability of live and dead cells to two fluorescent dyes (SYTO^{®} 10 and DEAD Red™). It was clearly observed that in the alginate:chitlac capsules more than 90% of the cells were living after 10 days in culture.

The nature of the macromolecular component of the ECM (Extra Cellular Matrix) synthesized in mixed capsules, and in those of alginate alone as control, was assessed by RT-PCR and biochemical assays. It emerged that the chondrocytes actively synthesize matrix and that the collagen component after various times in culture consists of type II collagen whereas that of the proteoglycans is represented by aggrecan, both being chondrogenic specific markers (figures 6 and 7).

To further demonstrate the proliferative capacity of these cells in the presence of the chitlac glycopolymer, [3^{H}]-thymidine incorporation within cells was measured. The chondrocytes were grown in the alginate capsules and alginate:chitlac capsules of example 12; 1µCi of [³H]-thymidine was introduced into the culture medium at relevant intervals (1 day, 5 days, 10 days and 15 days). The radioactivity thus incorporated was measured after 24 hours. The results show that at first days of culture, the replication is inhibited for the chondrocytes in the alginate capsules, whereas a rapid cell replication, which extends up to the first two weeks of culture, is observed for the condrocytes in the mixed (alginate:chitlac) capsules (figure 8).

These results were re-confirmed with optical microscope analyses on cells encapsulated as in example 12 and dyed with two staining protocols, one with toluidine blue and the other silver impregnation. Toluidine blue is a basic dye that as well as indicating cellular structures, interacts with GAGs, since it is negatively charged, and so highlighting their presence in the extracellular matrix. The images referring to the alginate capsules have a more intense colour due to the baseline signal of the negatively charged polymer. Silver impregnation instead enables collagen in the cellular matrix to be identified. Optical microscope photographs show a dark halo surrounding the cells which corresponds to collagen and also a violet-coloured halo which correspond to GAGs, confirming that the cells are continuing to synthesize matrix.

From the tissue engineering viewpoint, the experimental results given herein provide data relative to the planning and preparation of biocompatible three-dimensional matrices starting from binary aqueous solutions of anionic polysaccharides and modified cationic polysaccharides, such as oligosaccharide derivatives of chitosan. In a preferred embodiment these are respectively alginate and the lactose derivative of chitosan. The first is a biocompatible polymer with little or no capacity to generate a biological response but able to form gels in adequate conditions, and the second is a bioactive polymer unable in itself to form three-dimensional gels, but able to stimulate cell proliferation while simultaneously maintaining ECM synthesis capacity. At the same time this three-dimensional scaffold system of mixed composition is a method for culturing chondrocytes which ensures phenotype is maintained while simultaneously enabling their rapid expansion.

The experimental results presented hence demonstrate that the 3D matrices of the invention fulfil their purposes and can be usefully employed in the biomedical field and in particular for microencapsulation of cells i.e. that 3D matrices containing cells can be used in tissue engineering. Indeed, the results can be extended to 3D matrices for the microencapsulation of all cell types, whether isolated or in multicellular associations, used for tissue engineering, such as, by way of non-limiting example, chondrocytes, hepatocytes, pancreatic beta cells and islets of Langerhans, mesenchymal stem cells, endothelial cells, osteoblasts, keratinocytes.

Said results can also be extended to the encapsulation of drugs or pharmacologically active molecules, for the provision of delayed or controlled release systems of said compounds. In these cases, the preparation process is the same as aforesaid for encapsulation of cells or multicellular associations by simply substituting the cell suspension with the pharmacologically active molecules dissolved or suspended in the polymer solution.

## Claims

1. Compositions consisting of hydrogels **characterised in that** they are obtainable from aqueous solutions of mixtures of at least one lyotropic or thermotropic anionic polysaccharide and at least one derivative of chitosan with oligosaccharides, wherein said chitosan derivatives have a degree of derivatization of at least 40% and wherein said aqueous solutions of polysaccharide mixtures have an ionic strength of at least 50 mM and not greater than 175 mM and a pH of at least 7, by treating said aqueous solutions of polysaccharide mixtures with chemical or physical agents capable of gelling the lyotropic or thermotropic polyanionic polysaccharides comprised within the mixtures themselves.

2. Compositions consisting of hydrogels as claimed in claim 1, wherein the oligosaccharide derivatives of chitosan have a degree of derivatization of between 50% and 80%.

3. Compositions consisting of hydrogels as claimed in claim 2, wherein the oligosaccharide derivatives of chitosan have a degree of derivatization of 70%.

4. Compositions consisting of hydrogels as claimed in claim 1, wherein the oligosaccharide derivatives of chitosan are obtainable from the derivatization of chitosan with oligosaccharides comprising from 1 to 4 glycoside units.

5. Compositions consisting of hydrogels as claimed in claim 1, wherein the oligosaccharide derivatives of chitosan are obtainable from the derivatization of chitosan with oligosaccharides comprising from 2 to 4 glycoside units.

6. Compositions consisting of hydrogels as claimed in claim 5, wherein the oligosaccharides are chosen from the group consisting of lactose, cellobiose, cellotriose, maltose, maltotriose, maltotetraose, chitobiose, chitotriose, melibiose.

7. Compositions consisting of hydrogels as claimed in claim 6, wherein the oligosaccharide is lactose.

8. Compositions consisting of hydrogels as claimed in claims 4-7, wherein the chitosan has an average molecular weight up to 1,500 kDA.

9. Compositions consisting of hydrogels as claimed in claim 8, wherein the chitosan has an average molecular weight of between 400 kDa and 1,000 kDa.

10. Compositions consisting of hydrogels as claimed in claim 1, wherein the lyotropic anionic polysaccharides are selected from the group consisting of carrageenans, pectates and pectinates, alginates, gellan, rhamsan, welan, xanthan.

11. Compositions consisting of hydrogels as claimed in claim 10, wherein the lyotropic anionic polysaccharide is alginate.

12. Compositions consisting of hydrogels as claimed in claim 1, wherein the thermotropic anionic polysaccharides are chosen from the group consisting of partially sulphated agarose, carrageenan, cellulose sulphate; gellan, rhamsan, welan, xanthan.

13. Compositions consisting of hydrogels as claimed in claims 10-12, wherein the anionic polysaccharides have an average molecular weight up to 2,000 kDa.

14. Compositions consisting of hydrogels as claimed in claim 13, wherein the anionic polysaccharides have an average molecular weight of between 100 kDa and 1,000 kDa.

15. Compositions consisting of hydrogels as claimed in claim 14, wherein the anionic polysaccharides have an average molecular weight of 200 kDa.

16. Compositions consisting of hydrogels as claimed in claim 1, wherein the mixtures of at least one lyotropic or thermotropic anionic polysaccharide and at least one oligosaccharide derivative of chitosan comprise the anionic polysaccharides and oligosaccharide derivatives of chitosan in a weight ratio range of anionic polysaccharides to oligosaccharide derivatives of chitosan in a range from 3:1 to 1:1.

17. Compositions consisting of hydrogels as claimed in claim 1, wherein the aqueous solution of mixtures of at least one lyotropic or thermotropic anionic polysaccharide and at least one oligosaccharide derivative of chitosan have polymer concentrations up to 3% w/v.

18. Compositions consisting of hydrogels as claimed in claim 17, wherein the aqueous solutions of mixtures of at least one lyotropic or thermotropic anionic polysaccharide and at least one oligosaccharide derivative of chitosan have polymer concentrations in a range comprised from 1, 5% to 3% w/v.

19. Compositions consisting of hydrogels as claimed in claim 18, wherein the aqueous solutions of mixtures of at least one lyotropic or thermotropic anionic polysaccharide and at least one oligosaccharide derivative of chitosan have a polymer concentration of 2% w/v.

20. Compositions consisting of hydrogels as claimed in claim 1, wherein the aqueous solutions of mixtures of at least one lyotropic or thermotropic anionic polysaccharide and at least one oligosaccharide derivative of chitosan have a pH comprised in a range from 7 to 8.

21. Compositions consisting of hydrogels as claimed in claim 1, wherein the aqueous solutions of mixtures of at least one lyotropic or thermotropic anionic polysaccharide and at least one oligosaccharide derivative of chitosan have an ionic strength of 150 mM.

22. Compositions consisting of hydrogels as claimed in claim 1, wherein the x ionic strength of the said polysaccharide aqueous solutions is obtainable by means of addition of NaCl to obtain concentrations thereof in said aqueous solutions in a range comprised from 0.05 M and 0.175 M.

23. Compositions consisting of hydrogels as claimed in claim 21, wherein the ionic strength of the said polysaccharide aqueous solutions is obtainable by means of addition of NaCl to obtain concentrations thereof in said aqueous solutions of 0.15 M:

24. Compositions consisting of hydrogels as claimed in claim 1, wherein the x aqueous solutions of mixtures of at least one lyotropic or thermotropic anionic polysaccharide and at least one oligosaccharide derivative of chitosan have an osmolarity in a range comprised from 250 mM to 350 mM by a further addition of non-ionic solutes.

25. Compositions consisting of hydrogels as claimed in claim 1, wherein the chemical agents capable to gel the lyotropic anionic polysaccharides are aqueous solutions of monovalent, divalent or trivalent ions having a concentration higher than 10 mM.

26. Compositions consisting of hydrogels as claimed in claim 25, wherein X when the gelling agents are aqueous solutions of monovalent ions, they are selected from the group consisting of potassium, rubidium, caesium, thallium, silver and mixtures thereof.

27. Compositions consisting of hydrogels as claimed in claim 25, wherein when the gelling agents are aqueous solutions of divalent ions, they are selected from the group consisting of calcium, barium, strontium, copper, lead, magnesium, manganese and zinc and mixtures thereof with the proviso that the divalent cation is not magnesium when the anionic polysaccharide is alginate or pectate.

28. Compositions consisting of hydrogels as claimed in claim 25, wherein when the gelling agents are aqueous solutions of trivalent ions, they are chosen from the group consisting of aluminium, iron, gadolinium, therbium, europium and mixtures thereof.

29. Compositions consisting of hydrogels as claimed in claim 25, wherein the aqueous solutions of monovalent, divalent or trivalent ions have a concentration in a range comprised from 10 mM to 100 mM.

30. Compositions consisting of hydrogels as claimed in claim 29, wherein the aqueous solutions of monovalent, divalent or trivalent ions have a concentration of 50 mM or 100 mM.

31. Compositions consisting of hydrogels as claimed in claims 25-30, wherein the aqueous solutions of monovalent, divalent or trivalent ions have an osmolarity up to 0.3 M obtainable for further addition of ionic osmolites or non-ionic osmolites.

32. Compositions consisting of hydrogels as claimed in claim 1, wherein the physical agents capable to gel the thermotropic anionic polysaccharides are temperatures not higher than 40°C and not lower than 10°C.

33. Compositions consisting of hydrogels as claimed in claim 32, wherein the physical agents capable to gel the thermotropic anionic polysaccharides is a temperature of 20°C.

34. Compositions consisting of hydrogels as claimed in any one of the claims 1-33 further incorporating cells and/or active compounds.

35. Compositions consisting of hydrogels obtainable by a preparation process comprising at least the following steps:
a) preparing an aqueous solution of a mixture of at least one lyotropic or thermotropic anionic polysaccharide and at least one derivative of chitosan with oligosaccharides, said chitosan derivatives having a degree of derivatisation of at least 40% and the aqueous solutions having an ionic strength of at least 50 mM and not greater than 175 mM and a pH of at least 7, then optionally adding cell's and/or active compounds to the prepared polysaccharide solutions;
b) adding the solution prepared in step a) by a suitable means for obtaining the required hydrogel form, to a gelling solution either containing the crosslinking ion for lyotropic anionic polysaccharides or being at a suitable temperature for the thermotropic anionic polysaccharides;
c) removing the formed hydrogel, optionally incorporating the cells and/or active compounds, by suitable methods.

36. Compositions consisting of hydrogels as claimed in any one of the claims 2-34 wherein said hydrogels are obtainable by a preparation process according to claim 35.

37. Process for preparing hydrogels comprising at least the following steps:
a) preparing an aqueous solution of a mixture of at least one lyotropic or thermotropic anionic polysaccharide and at least one derivative of chitosan with oligosacchanides, said chitosan derivatives having a degree of derivatization of at least 40% and the aqueous solutions having an ionic strength of at least 50 mM and not greater than 175 mM and a pH of at least 7, then optionally adding cells and/or active compounds to the prepared polysaccharide solutions;
b) adding the solution prepared in step a) by a suitable means for obtaining the required hydrogel form, to a gelling solution either containing the crosslinking ion for lyotropic anionic polysaccharides or being at a suitable temperature for the thermotropic anionic polysaccharides;
c) removing the formed hydrogel, optionally incorporating cells and/or active compounds, by suitable methods.

38. Process as claimed in claim 37 for preparing the hydrogels claimed in claims 2-34.

39. Process for preparing hydrogels as claimed in claim 38, wherein the hydrogels have microcapsule, cylindrical or discoidal form.

40. Use of hydrogels claimed in any one of the claims 1-36 for applications in the human and non-human biomedical field.

41. Use of hydrogels claimed ih claim 40 incorporating cells for use in tissue engineering.

42. Use of hydrogels claimed in claim 40 incorporating active compounds for use in the delayed or controlled release of said compounds.

## Patentansprüche

1. Zusammensetzungen bestehend aus Hydrogelen, **dadurch gekennzeichnet, dass** diese aus wässrigen Lösungen von Mischungen aus wenigstens einem lyotropen oder thermotropen anionischen Polysaccharid und wenigstens einem Derivat von Chitosan mit Oligosacchariden, wobei die Chitosanderivate einen Derivatisierungsgrad von wenigstens 40 % aufweisen, und, wobei die wässrigen Lösungen aus Polysaccharidmischungen eine Ionenstärke von wenigstens 50 mM und von nicht größer als 175 mM sowie einen pH-Wert von wenigstens 7 aufweisen, erhältlich sind durch Behandeln der wässrigen Lösungen von Polysaccharidmischungen mit chemischen oder physikalischen Mitteln, welche die in den Mischungen selbst enthaltenen lyotropen oder thermotropen polyanionischen Polysaccharide gelieren können.

2. Zusammensetzungen bestehend aus Hydrogelen nach Anspruch 1, wobei die Oligosaccharidderivate von Chitosan einen Derivatisierungsgrad zwischen 50 % und 80 % aufweisen.

3. Zusammensetzungen bestehend aus Hydrogelen nach Anspruch 2, wobei die Oligosaccharidderivate von Chitosan einen Derivatisierungsgrad von 70 % aufweisen.

4. Zusammensetzungen bestehend aus Hydrogelen nach Anspruch 1, wobei die Oligosaccharidderivate von Chitosan durch Derivatisierung von Chitosan mit Oligosacchariden, welche 1 bis 4 Glycosideinheiten aufweisen, erhältlich sind.

5. Zusammensetzungen bestehend aus Hydrogelen nach Anspruch 1, wobei die Oligosaccharidderivate von Chitosan durch Derivatisierung von Chitosan mit Oligosacchariden, welche 2 bis 4 Glycosideinheiten aufweisen, erhältlich sind.

6. Zusammensetzungen bestehend aus Hydrogelen nach Anspruch 5, wobei die Oligosaccharide aus der Gruppe ausgewählt sind, welche aus Lactose, Cellobiose, Cellotriose, Maltose, Maltotriose, Maltotetraose, Chitobiose, Chitotriose und Melibiose besteht.

7. Zusammensetzungen bestehend aus Hydrogelen nach Anspruch 6, wobei das Oligosaccharid Lactose ist.

8. Zusammensetzungen bestehend aus Hydrogelen nach einem der Ansprüche 4 bis 7, wobei das Chitosan ein durchschnittliches Molekulargewicht von bis zu 1.500 kDa aufweist.

9. Zusammensetzungen bestehend aus Hydrogelen nach Anspruch 8, wobei das Chitosan ein durchschnittliches Molekulargewicht zwischen 400 kDa und 1.000 kDa aufweist.

10. Zusammensetzungen bestehend aus Hydrogelen nach Anspruch 1, wobei die lyotropen anionischen Polysaccharide aus der Gruppe ausgewählt sind, welche aus Carrageenanen, Pektaten und Pektinaten, Alginaten, Gellan, Rhamsan, Welan und Xanthan besteht.

11. Zusammensetzungen bestehend aus Hydrogelen nach Anspruch 10, wobei das lyotrope anionische Polysaccharid Alginat ist.

12. Zusammensetzungen bestehend aus Hydrogelen nach Anspruch 1, wobei die thermotropen anionischen Polysaccharide aus der Gruppe ausgewählt sind, welche aus partiell sulfatisierter Agarose, Carrageenanen, Cellulosesulfat, Gellan, Rhamsan, Welan und Xanthan besteht.

13. Zusammensetzungen bestehend aus Hydrogelen nach einem der Ansprüche 10 bis 12, wobei die anionischen Polysaccharide ein durchschnittliches Molekulargewicht von bis zu 2.000 kDa aufweisen.

14. Zusammensetzungen bestehend aus Hydrogelen nach Anspruch 13, wobei die anionischen Polysaccharide ein durchschnittliches Molekulargewicht zwischen 100 kDa und 1.000 kDa aufweisen.

15. Zusammensetzungen bestehend aus Hydrogelen nach Anspruch 14, wobei die anionischen Polysaccharide ein durchschnittliches Molekulargewicht von 200 kDa aufweisen.

16. Zusammensetzungen bestehend aus Hydrogelen nach Anspruch 1, wobei die Mischungen aus wenigstens einem lyotropen oder thermotropen anionischen Polysaccharid und wenigstens einem Oligosaccharidderivat von Chitosan die anionischen Polysaccharide und Oligosaccharidderivate von Chitosan in einem Gewichtsverhältnisbereich von anionischen Polysacchariden zu Oligosaccharidderivaten von Chitosan in einem Bereich von 3:1 bis 1:1 enthalten.

17. Zusammensetzungen bestehend aus Hydrogelen nach Anspruch 1, wobei die wässrigen Lösungen von Mischungen aus wenigstens einem lyotropen oder thermotropen anionischen Polysaccharid und wenigstens einem Oligosaccharidderivat von Chitosan Polymerkonzentrationen von bis zu 3 % (Gew./Vol.) aufweisen.

18. Zusammensetzungen bestehend aus Hydrogelen nach Anspruch 17, wobei die wässrigen Lösungen von Mischungen aus wenigstens einem lyotropen oder thermotropen anionischen Polysaccharid und wenigstens einem Oligosaccharidderivat von Chitosan Polymerkonzentrationen in einem Bereich zwischen 1.5 % und 3 % (Gew./Vol.) aufweisen.

19. Zusammensetzungen bestehend aus Hydrogelen nach Anspruch 18, wobei die wässrigen Lösungen von Mischungen aus wenigstens einem lyotropen oder thermotropen anionischen Polysaccharid und wenigstens einem Oligosaccharidderivat von Chitosan eine Polymerkonzentration von 2 % (Gew./Vol.) aufweisen.

20. Zusammensetzungen bestehend aus Hydrogelen nach Anspruch 1, wobei die wässrigen Lösungen von Mischungen aus wenigstens einem lyotropen oder thermotropen anionischen Polysaccharid und wenigstens einem Oligosaccharidderivat von Chitosan einen pH-Wert in einem Bereich zwischen 7 und 8 aufweisen.

21. Zusammensetzungen bestehend aus Hydrogelen nach Anspruch 1, wobei die wässrigen Lösungen von Mischungen aus wenigstens einem lyotropen oder thermotropen anionischen Polysaccharid und wenigstens einem Oligosaccharidderivat von Chitosan eine Ionenstärke von 150 mM aufweisen.

22. Zusammensetzungen bestehend aus Hydrogelen nach Anspruch 1, wobei die Ionenstärke der wässrigen Polysaccharidlösungen durch die Zugabe von NaCl, um Konzentrationen hiervon in den wässrigen Lösungen in einem Bereich zwischen 0,05 M und 0,175 M zu erhalten, erhältlich ist.

23. Zusammensetzungen bestehend aus Hydrogelen nach Anspruch 21, wobei die Ionenstärke der wässrigen Polysaccharidlösungen durch die Zugabe von NaCl, um Konzentrationen hiervon in den wässrigen Lösungen von 0,15 M zu erhalten, erhältlich ist.

24. Zusammensetzungen bestehend aus Hydrogelen nach Anspruch 1, wobei die wässrigen Lösungen von Mischungen aus wenigstens einem lyotropen oder thermotropen anionischen Polysaccharid und wenigstens einem Oligosaccharidderivat von Chitosan durch die weitere Zugabe von nicht ionischen gelösten Stoffen eine Osmolarität in einem Bereich zwischen 250 mM und 350 mM aufweisen.

25. Zusammensetzungen bestehend aus Hydrogelen nach Anspruch 1, wobei die chemischen Mittel, welche die lyotropen anionischen Polysaccharide gelieren können, wässrige Lösungen aus monovalenten, divalenten oder trivalenten Ionen mit einer Konzentration von mehr als 10 mM sind.

26. Zusammensetzungen bestehend aus Hydrogelen nach Anspruch 25, wobei, wenn die Gelierungsmittel wässrige Lösung aus monovalenten Ionen sind, diese aus der Gruppe ausgewählt sind, welche aus Kalium, Rubidium, Cäsium, Thallium, Silber und Mischungen hiervon besteht.

27. Zusammensetzungen bestehend aus Hydrogelen nach Anspruch 25, wobei, wenn die Gelierungsmittel wässrige Lösungen aus divalenten Ionen sind, diese aus der Gruppe ausgewählt sind, welche aus Calcium, Barium, Strontium, Kupfer, Blei, Magnesium, Mangan und Zink sowie Mischungen hiervon besteht, mit der Maßgabe, dass das divalente Kation nicht Magnesium ist, wenn das anionische Polysaccharid Alginat oder Pektat ist.

28. Zusammensetzungen bestehend aus Hydrogelen nach Anspruch 25, wobei, wenn die Gelierungsmittel wässrige Lösungen aus trivalenten Ionen sind, diese aus der Gruppe ausgewählt sind, welche aus Aluminium, Eisen, Gadolinium, Therbium, Europium und Mischungen hiervon besteht.

29. Zusammensetzungen bestehend aus Hydrogelen nach Anspruch 25, wobei die wässrigen Lösungen aus monovalenten, divalenten oder trivalenten Ionen eine Konzentration in einem Bereich zwischen 10 mM und 100 mM aufweisen.

30. Zusammensetzungen bestehend aus Hydrogelen nach Anspruch 29, wobei die wässrigen Lösungen aus monovalenten, divalenten oder trivalenten Ionen eine Konzentration von 50 mM oder 100 mM aufweisen.

31. Zusammensetzungen bestehend aus Hydrogelen nach einem der Ansprüche 25 bis 30, wobei die wässrigen Lösungen aus monovalenten, divalenten oder trivalenten Ionen durch die weitere Zugabe von ionischen Osmolyten oder nicht ionischen Osmolyten eine Osmolarität von bis zu 0,3 M erhältlich aufweisen.

32. Zusammensetzungen bestehend aus Hydrogelen nach Anspruch 1, wobei die physikalischen Mittel, welche die thermotropen anionischen Polysaccharide gelieren können, Temperaturen von nicht größer als 40°C und von nicht weniger als 10°C sind.

33. Zusammensetzungen bestehend aus Hydrogelen nach Anspruch 32, wobei das physikalische Mittel, welches die thermotropen anionischen Polysaccharide gelieren kann, eine Temperatur von 20°C ist.

34. Zusammensetzungen bestehend aus Hydrogelen nach einem der Ansprüche 1 bis 33, welche des Weiteren Zellen und/oder aktive Bestandteile enthalten.

35. Zusammensetzungen bestehend aus Hydrogelen erhältlich durch ein Herstellungsverfahren, welches wenigstens die nachfolgenden Schritte umfasst:
a) Herstellen einer wässrigen Lösung von einer Mischung aus wenigstens einem lyotropen oder thermotropen anionischen Polysaccharid und wenigstens einem Oligosaccharidderivat von Chitosan, wobei die Chitosanderivate einen Derivatisierungsgrad von wenigstens 40 % aufweisen und die wässrigen Lösungen eine Ionenstärke von wenigstens 50 mM und nicht mehr als 175 mM sowie einen pH-Wert von wenigstens 7 aufweisen, und dann optional Zugabe von Zellen und/oder von aktiven Bestandteilen zu den hergestellten Polysaccharidlösungen,
b) Zugabe der in dem Schritt a) hergestellten Lösung durch geeignete Mittel zum Erhalten der benötigten Hydrogelform zu einer Gelierlösung, welche entweder ein Vernetzungsion für lyotrope anionische Polysaccharide enthält oder sich bei einer geeigneten Temperatur für die thermotropen anionischen Polysaccharide befindet,
c) Entfernen des gebildeten Hydrogels und optional Einbau von Zellen und/oder von aktiven Verbindungen durch geeignete Verfahren.

36. Zusammensetzungen bestehend aus Hydrogelen nach einem der Ansprüche 2 bis 34, wobei die Hydrogele durch ein Herstellungsverfahren nach Anspruch 35 erhältlich sind.

37. Verfahren zum Herstellen von Hydrogelen, welches wenigstens die nachfolgenden Schritte umfasst:
a) Herstellen einer wässrigen Lösung von einer Mischung aus wenigstens einem lyotropen oder thermotropen anionischen Polysaccharid und wenigstens einem Derivat von Chitosan mit Oligosacchariden, wobei die Chitosanderivate einen Derivatisierungsgrad von wenigstens 40 % aufweisen und die wässrigen Lösungen eine Ionenstärke von wenigstens 50 mM und nicht mehr als 175 mM sowie einen pH-Wert von wenigstens 7 aufweisen, und dann optional Zugabe von Zellen und/oder von aktiven Bestandteilen zu den hergestellten Polysaccharidlösungen,
b) Zugabe der Lösung, welche in dem Schritt a) hergestellt worden ist, durch geeignete Mittel zum Erhalten der erforderlichen Hydrogelform zu einer Gelierlösung, welche entweder ein Vernetzungsion für lyotrope anionische Polysaccharide enthält oder sich bei einer geeigneten Temperatur für die thermotropen anionischen Polysaccharide befindet,
c) Entfernen des gebildeten Hydrogels und optional Einbau von Zellen und/oder von aktiven Bestandteilen durch geeignete Verfahren.

38. Verfahren nach Anspruch 37 zum Herstellen der in einem der Ansprüche 2 bis 34 beanspruchten Hydrogele.

39. Verfahren zum Herstellen von Hydrogelen nach Anspruch 38, wobei die Hydrogele die Form einer Mikrokapsel, eine zylindrische Form oder eine diskoidale Form aufweisen.

40. Verwendung von Hydrogelen nach einem der Ansprüche 1 bis 36 bei Anwendungen auf dem humanen oder nicht humanen biomedizinischen Gebiet.

41. Verwendung von Hydrogelen nach Anspruch 40 zum Einbau von Zellen zur Verwendung bei der Gewebezüchtung.

42. Verwendung von Hydrogelen nach Anspruch 40 zum Einbau von aktiven Bestandteilen zur Verwendung in der verzögerten oder kontrollierten Freisetzung der Verbindungen.

## Revendications

1. Compositions constituées d'hydrogels, **caractérisées en ce qu'**elles peuvent être obtenues à partir de solutions aqueuses de mélanges constitués d'au moins un polysaccharide anionique lyotrope ou thermotrope et d'au moins un dérivé de chitosane avec des oligosaccharides, dans lesquelles lesdits dérivés de chitosane ont un degré de dérivatisation d'au moins 40 % et dans lesquelles lesdites solutions aqueuses de mélanges de polysaccharides ont une force ionique d'au moins 50 mM et non supérieure à 175 mM et un pH d'au moins 7, en traitant lesdites solutions aqueuses de mélanges de polysaccharides avec des agents chimiques ou physiques capables de gélifier les polysaccharides polyanioniques lyotropes ou thermotropes inclus dans les mélanges eux-mêmes.

2. Compositions constituées d'hydrogels selon la revendication 1, dans lesquelles les dérivées d'oligosaccharides du chitosane ont un degré de dérivatisation entre 50 % et 80 %.

3. Compositions constituées d'hydrogels selon la revendication 2, dans lesquelles les dérivées d'oligosaccharides du chitosane ont un degré de dérivatisation de 70 %.

4. Compositions constituées d'hydrogels selon la revendication 1, dans lesquelles les dérivés d'oligosaccharides du chitosane peuvent être obtenus à partir de la dérivatisation du chitosane avec des oligosaccharides comportant de 1 à 4 unités glycosidiques.

5. Compositions constituées d'hydrogels selon la revendication 1, dans lesquelles lesdits dérivés d'oligosaccharides du chitosane peuvent être obtenus à partir de la dérivatisation du chitosane avec des oligosaccharides comportant de 2 à 4 unités glycosidiques.

6. Compositions constituées d'hydrogels selon la revendication 5, dans lesquelles les oligosaccharides sont choisis parmi le groupe constitué de lactose, de cellobiose, de cellotriose, de maltose, de maltotriose, de maltotétraose, de chitobiose, de chitotriose, de mélibiose.

7. Compositions constituées d'hydrogels selon la revendication 6, dans lesquelles l'oligosaccharide est le lactose.

8. Compositions constituées d'hydrogels selon les revendications 4 à 7, dans lesquelles le chitosane a un poids moléculaire moyen allant jusqu'à 1 500 kDa.

9. Compositions constituées d'hydrogels selon la revendication 8, dans lesquelles le chitosane a un poids moléculaire moyen entre 400 kDa et 1 000 kDa.

10. Compositions constituées d'hydrogels selon la revendication 1, dans lesquelles les polysaccharides anioniques lyotropes sont choisis parmi le groupe constitué de carraghénines, de pectates et de pectinates, d'alginates, de gellan, de rhamsan, de wélan, de xanthane.

11. Compositions constituées d'hydrogels selon la revendication 10, dans lesquelles le polysaccharide anionique lyotrope est l'alginate.

12. Compositions constituées d'hydrogels selon la revendication 1, dans lesquelles les polysaccharides anioniques thermotropes sont choisis parmi le groupe constitué d'agarose partiellement sulfaté, de carraghénine, de sulfate de cellulose, de gellan, de rhamsan, de wélan, de xanthane.

13. Compositions constituées d'hydrogels selon les revendications 10 à 12, dans lesquelles les polysaccharides anioniques ont un poids moléculaire moyen allant jusqu'à 2 000 kDa.

14. Compositions constituées d'hydrogels selon la revendication 13, dans lesquelles les polysaccharides anioniques ont un poids moléculaire moyen entre 100 kDa et 1 000 kDa.

15. Compositions constituées d'hydrogels selon la revendication 14, dans lesquelles les polysaccharides anioniques ont un poids moléculaire moyen de 200 kDa.

16. Compositions constituées d'hydrogels selon la revendication 1, dans lesquelles les mélanges constitués d'au moins un polysaccharide anionique lyotrope ou thermotrope et d'au moins un dérivé d'oligosaccharide du chitosane comportent les polysaccharides anioniques et les dérivés d'oligosaccharide du chitosane dans une plage de rapport de poids polysaccharides anioniques / dérivés d'oligosaccharide dans une plage de 3:1 à 1:1.

17. Compositions constituées d'hydrogels selon la revendication 1, dans lesquelles les solutions aqueuses de mélanges d'au moins un polysaccharide anionique lyotrope ou thermotrope et d'au moins un dérivé d'oligosaccharide du chitosane ont des concentrations en polymère allant jusqu'à 3 % p/v.

18. Compositions constituées d'hydrogels selon la revendication 17, dans lesquelles les solutions aqueuses de mélanges d'au moins un polysaccharide anionique lyotrope ou thermotrope et d'au moins un dérivé d'oligosaccharide du chitosane ont des concentrations en polymère dans une plage comprise entre 1.5 % et 3 % p/v.

19. Compositions constituées d'hydrogels selon la revendication 18, dans lesquelles les solutions aqueuses de mélanges d'au moins un polysaccharide anionique lyotrope ou thermotrope et d'au moins un dérivé d'oligosaccharide du chitosane ont une concentration en polymère de 2 % p/v.

20. Compositions constituées d'hydrogels selon la revendication 1, dans lesquelles les solutions aqueuses de mélanges d'au moins un polysaccharide anionique lyotrope ou thermotrope et d'au moins un dérivé d'oligosaccharide du chitosane ont un pH compris dans une plage de 7 à 8.

21. Compositions constituées d'hydrogels selon la revendication 1, dans lesquelles les solutions aqueuses de mélanges d'au moins un polysaccharide anionique lyotrope ou thermotrope et d'au moins un dérivé d'oligosaccharide du chitosane ont une force ionique de 150 mM.

22. Compositions constituées d'hydrogels selon la revendication 1, dans lesquelles la force ionique desdites solutions aqueuses de polysaccharides peut être obtenue par l'ajout de NaCl afin d'obtenir des concentrations de celui-ci dans lesdites solutions aqueuses dans une plage comprise entre 0,05 M et 0,175 M.

23. Compositions constituées d'hydrogels selon la revendication 21, dans lesquelles la force ionique desdites solutions aqueuses de polysaccharides peut être obtenue par l'ajout de NaCl afin d'obtenir des concentrations de celui-ci dans lesdites solutions aqueuses de 0,15 M.

24. Compositions constituées d'hydrogels selon la revendication 1, dans lesquelles les solutions aqueuses de mélanges d'au moins un polysaccharide anionique lyotrope ou thermotrope et d'au moins un dérivé d'oligosaccharide du chitosane ont une osmolarité dans une plage comprise entre 250 mM et 350 mM par un ajout supplémentaire de solutés non ioniques.

25. Compositions constituées d'hydrogels selon la revendication 1, dans lesquelles les agents chimiques capables de gélifier les polysaccharides anioniques lyotropes sont des solutions aqueuses d'ions monovalents, divalents ou trivalents ayant une concentration supérieure à 10 mM.

26. Compositions constituées d'hydrogels selon la revendication 25, dans lesquelles lorsque les agents gélifiants sont des solutions aqueuses d'ions monovalents, ils sont choisis parmi le groupe constitué du potassium, du rubidium, du césium, du thallium, de l'argent et de mélanges de ceux-ci.

27. Compositions constituées d'hydrogels selon la revendication 25, dans lesquelles lorsque les agents gélifiants sont des solutions aqueuses d'ions divalents, ils sont choisis parmi le groupe constitué du calcium, du baryum, du strontium, du cuivre, du plomb, du magnésium, du manganèse et du zinc et de mélanges de ceux-ci, à la condition que le cation divalent ne soit pas le magnésium lorsque le polysaccharide anionique est l'alginate ou le pectate.

28. Compositions constituées d'hydrogels selon la revendication 25, dans lesquelles lorsque les agents gélifiants sont des solutions aqueuses d'ions trivalents, ils sont choisis parmi le groupe constitué de l'aluminium, du fer, du gadolinium, du terbium, de l'europium et de mélanges de ceux-ci.

29. Compositions constituées d'hydrogels selon la revendication 25, dans lesquelles les solutions aqueuses d'ions monovalents, divalents ou trivalents ont une concentration dans une plage comprise entre 10 mM et 100 mM.

30. Compositions constituées d'hydrogels selon la revendication 29, dans lesquelles les solutions aqueuses d'ions monovalents, divalents ou trivalents ont une concentration de 50 mM ou 100 mM.

31. Compositions constituées d'hydrogels selon les revendications 25 à 30, dans lesquelles les solutions aqueuses d'ions monovalents, divalents ou trivalents ont une osmolarité allant jusqu'à 0,3 M pouvant être obtenue pour l'ajout supplémentaire d'osmolites ioniques ou d'osmolites non ioniques.

32. Compositions constituées d'hydrogels selon la revendication 1, dans lesquelles les agents physiques capables de gélifier les polysaccharides anioniques thermotropes sont des températures non supérieures à 40°C et non inférieures à 10°C.

33. Compositions constituées d'hydrogels selon la revendication 32, dans lesquelles les agents physiques capables de gélifier les polysaccharides anioniques thermotropes sont une température de 20°C.

34. Compositions constituées d'hydrogels selon l'une quelconque des revendications 1 à 33, incorporant également des cellules et/ou des composés actifs.

35. Compositions constituées d'hydrogels pouvant être obtenues par un procédé de préparation comportant au moins les étapes suivantes consistant à :
a) préparer une solution aqueuse d'un mélange constitué d'au moins un polysaccharide anionique lyotrope ou thermotrope et d'au moins un dérivé d'oligosaccharide de chitosane, lesdits dérivés de chitosane ayant un degré de dérivatisation d'au moins 40 % et les solutions aqueuses ayant une force ionique d'au moins 50 mM et non supérieure à 175 mM et un pH d'au moins 7, puis ajouter facultativement des cellules et/ou des composés actifs aux solutions de polysaccharides préparées,
b) ajouter la solution préparée à l'étape a) par l'intermédiaire de moyens adaptés pour obtenir la forme d'hydrogel requise, à une solution de gélification soit contenant l'ion de réticulation pour des polysaccharides anioniques lyotropes, soit étant à une température adaptée pour les polysaccharides anioniques thermotropes,
c) retirer l'hydrogel formé, en incorporant facultativement les cellules et/ou les composés actifs, par des procédés adaptés.

36. Compositions constituées d'hydrogels selon l'une quelconque des revendications 2 à 34, dans lesquelles lesdits hydrogels peuvent être obtenus par un procédé de préparation selon la revendication 35.

37. Procédé pour préparer des hydrogels comportant au moins les étapes suivantes consistant à :
a) préparer une solution aqueuse d'un mélange constitué d'au moins un polysaccharide anionique lyotrope ou thermotrope et d'au moins un dérivé de chitosane avec des oligosaccharides, lesdits dérivés de chitosane ayant un degré de dérivatisation d'au moins 40 % et les solutions aqueuses ayant une force ionique d'au moins 50 mM et non supérieure à 175 mM et un pH d'au moins 7, puis ajouter facultativement des cellules et/ou des composés actifs aux solutions de polysaccharides préparées,
b) ajouter la solution préparée à l'étape a) par l'intermédiaire de moyens adaptés pour obtenir la forme d'hydrogel requise, à une solution de gélification soit contenant l'ion de réticulation pour des polysaccharides anioniques lyotropes, soit étant à une température adaptée pour les polysaccharides anioniques thermotropes,
c) retirer l'hydrogel formé, en incorporant facultativement des cellules et/ou des composés actifs, par des procédés adaptés.

38. Procédé selon la revendication 37 pour préparer les hydrogels revendiqués aux revendications 2 à 34.

39. Procédé pour préparer des hydrogels tel que revendiqué à la revendication 38 dans lequel les hydrogels ont une forme de microcapsule, cylindrique ou discoïdale.

40. Utilisation d'hydrogels revendiqués dans l'une quelconque des revendications 1 à 36 pour des applications dans le domaine biomédical humain et non humain.

41. Utilisation d'hydrogels revendiquée dans la revendication 40 incorporant des cellules pour une utilisation en ingénierie tissulaire.

42. Utilisation d'hydrogels revendiquée à la revendication 40 incorporant des composés actifs pour une utilisation dans la libération retardée ou contrôlée desdits composés.
